# EUROPEAN PATENT APPLICATION

(11) **EP 4 471 009 A1**
(43) Date of publication of application: **04.12.2024**
(21) Application number: 23305869.2
(22) Date of filing: 01.06.2023
(51) Int. Cl.: C07C 329/02, C07C 333/08, C07F 7/08

(54) **PROCESS FOR TRAPPING THIOCARBONYL FLUORIDE**

(71) Applicant: Centre National de la Recherche Scientifique, 75016 Paris (FR); Institut National des Sciences Appliquées de Rouen Normandie, 76801 Saint-Etienne-du-Rouvray, Cedex (FR); Université de Rouen Normandie, 76130 Mont-Saint-Aignan (FR)
(72) Inventor: BESSET, Tatiana, 76230 ISNEAUVILLE (FR); CASTANHEIRO MATIAS, Thomas, 76770 MALAUNAY (FR); NOBILE, Enzo, 91300 MASSY (FR); ARRIBAT, Mathieu, 27460 Alizay (FR)
(74) Representative: Brevalex

(57) **Abstract**

The present invention relates to a process for trapping SCF₂ comprising the following steps:
- a step of reacting a compound A-SCF₃ with a nucleophile, in the presence of a metallic complex comprising nickel, a ligand, and a solvent,

A being selected from the group consisting of: (C₆-C₁₀)aryl groups, heteroaryl groups, and a vinyl compound, and
- a step of reacting SCF₂ with a compound RH, R being a hydrocarbon group, comprising optionally at least one heteroatom,
for obtaining a compound having the formula R-C(=S)-F.

## Description

The present invention concerns a process for trapping thiocarbonyl fluoride or analogues thereof.

In a society concerned about the environment, earth and aquatic systems pollution and circular economy, development of new technologies to meet such objectives is nowadays a real challenge and a hot topic across the whole chemical industry.

The field of organofluorine chemistry is unavoidable and fluorinated compounds are highly represented in many fields such as materials science, pharmaceutical and agrochemical industries. Indeed, the incorporation of a fluorine atom or a fluorinated unit can modify the physico-chemical properties of organic molecules in a significant way explaining the interest of the scientific community regarding this research area. However, the prevalence of these compounds raises the question of their fate and degradation. Recently, there has been a strong awareness from the scientific community and society regarding chlorofluorocarbons (CFCs), hydrofluorocarbons (HFCs) and per- and poly-fluoroalkyl substances (PFAs). It is now important to go even further and to look at the fate of emerging fluorinated groups that have become essential, such as derivatives containing a SCF₃ unit (eg. Toltrazuril and Fipronil).

In this context, the development of tools allowing to valorize these fluorinated derivatives, which, once used, are then considered as waste by converting them into compounds with high added value would have an undeniable ecological impact and allow for cost reduction. It is therefore essential to develop innovative tools to meet this challenge and to remove this synthetic lock.

There is currently no technology allowing to selectively cleave C(sp²)-SRf bonds on (hetero)aromatic derivatives or vinyl positions or C(sp³)-SRf bonds and any progress would have a major impact from a scientific and environmental point of view.

With a view to the circular economy, which is nowadays an essential part of industrial purposes, notably through the 3R logic (Reduce, Reuse, Recycle), there is a need of reusing the fluorinated residue released in the course of the de-fluorination reaction.

One aim of the present invention is thus to provide a process for trapping a fluorinated residue, thiocarbonyl fluoride (SCF₂), SCF₃⁻ or F⁻.

Thus, the present invention relates to a process for trapping SCF₂ comprising the following steps:
- a step of reacting a compound having the formula (I)

   A-SCF₃ (I)

   with a nucleophile, in the presence of a metallic complex comprising nickel, a ligand, and a solvent,
   A being selected from the group consisting of:
      . (C₆-C₁₀)aryl groups,
         said aryl groups being optionally substituted with at least one substituent preferably selected from the group consisting of: halogen, (C₁-C₆)alkyl, (C₁-C₆)alkoxy, -CN, -CF₃, -OCF₃, -NRₐR_{b}, -SiRₐR_{b}R_{c}, -BRₐR_{b}, (C₂-C₆)alkenyl, (C₂-C₆)alkynyl, -C(=O)-NRₐR_{b}, and -C(=O)-O(C₁-C₆)alkyl;
         Rₐ and R_{b} being independently from each other H or a (C₁-C₆)alkyl group; R_{c} being a (C₁-C₆)alkyl group;
      . heteroaryl groups comprising from 5 to 10 atoms and including at least one heteroatom selected from O, N, and S,
         said heteroaryl groups being optionally substituted with at least one substituent preferably selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
      . a vinyl compound having the following formula (III): 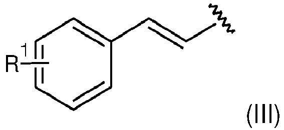 R¹ being selected from the group consisting of: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
   said step leading to the formation of a compound AH and SCF₂, and
      - a step of reacting SCF₂ obtained at the previous step with a compound RH, R being a hydrocarbon group, comprising optionally at least one heteroatom,
         for obtaining a compound having the formula R-C(=S)-F.

The present invention provides different processes for the valorization of the fluorinated residues *in situ* generated during a de-fluorination reaction. Those residues might exist under different forms including XSCF₃ (X being any couteranion such as Na or H), such as a HSCF₃, a highly volatile gas (bp = approx. -30°C), or as a thiocarbonyl fluoride in equilibrium with a fluoride anion. The HSCF₃, or thiocarbonyl fluoride/ F- can be trapped for easy disposal, or can react with other compounds to afford another fluorinated molecule of interest.

The present invention also relates to a process for trapping a fluorinated residue, such as HSCF₃, or the fluoride anion, comprising the following steps:
- a step of reacting a compound having the formula (I)

   A-SCF₃ (I)

   with a nucleophile, in the presence of a metallic complex comprising nickel, a ligand, and a solvent,
   A being as defined above,
   said step leading to the release of a fluorinated residue, such as HSCF₃, or the fluoride anion, and
- a step of reacting said fluorinated residue, such as HSCF₃, or the fluoride anion obtained at the previous step with a compound RH, R being a hydrocarbon group, comprising optionally at least one heteroatom.

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heteroaryl group" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

When an alkyl group is substituted with an aryl group, the term "arylalkyl" or "aralkyl" group is used. The "arylalkyl" or "aralkyl" groups are aryl-alkyl- groups, the aryl and alkyl groups being as defined above. Among the arylalkyl groups, mention may in particular be made of the benzyl or phenethyl groups.

The term "halogen" means: a fluorine, a chlorine, a bromine or an iodine.

The term "alkoxy group" means: an -O-alkyl group where the alkyl group is as previously defined. By way of examples, mention may be made of -O-(C₁-C₄)alkyl groups, and in particular the -O-methyl group, the -O-ethyl group, as -O-Csalkyl group, the -O-propyl group, the -O-isopropyl group, and as -O-C₄alkyl group, the -O-butyl,-O-isobutyl or -O-tert-butyl group.

The term "alkynyl" as employed herein includes unsaturated, nonaromatic, hydrocarbon groups having 2 to 6 carbons, and comprising at least one triple bond. Preferably, the alkynyl group is linear. Preferably, the alkynyl group is a -(CH₂)ₘ-C≡CH group, m being an integer comprised from 1 to 4.

The term "alkenyl" as employed herein includes unsaturated, nonaromatic, hydrocarbon groups having 2 to 6 carbons, and comprising at least one double bond. Preferably, the alkenyl group is linear. Preferably, the alkenyl group is a -(CH₂)ₘ-CH=CH₂ group, m being an integer comprised from 1 to 4.

The abovementioned "alkyl", "aryl", and "heteroaryl" groups can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "haloalkyl group" means: an alkyl group as defined above, in which one or more of the hydrogen atoms is(are) replaced with a halogen atom. By way of example, mention may be made of fluoroalkyls, in particular CF₃ or CHF₂.

The term "carboxyl" means: a COOH group.

The term "oxo" means: "=O".

In one embodiment, the compound RH is selected from the group consisting of: amines, alcohols, thiols, phosphines, phosphonates, Grignard reagents, R'Li, selenols, R'₂B(OH), and R'₃SiOH, R' representing an optionally substituted alkyl, aryl, vinyl, or alkynyl group, said alkyl, aryl, and alkynyl groups being as defined above.

In one embodiment, the compound RH is selected from the group consisting of: secondary alkyl amines, anilines, alkyl alcohols, alkyl thiols, aryl thiols, aryl selenols, phosphine oxides, aryl phosphines, and Grignard reagents.

Preferably, the compound RH is selected from the group consisting of: HNR_{d}Rₑ, and OR_{f}, R_{d} and Rₑ being independently selected from the group consisting of: (C₁-C₆)alkyl groups and (C₆-C₁₀)aryl groups; and R_{f} being a (C₁-C₆)alkyl group or a (C₆-C₁₀)aryl(C₁-C₆)alkyl group.

In one embodiment of the process according to the invention, the nucleophile is an hydride.

In one embodiment of the process according to the invention, the nucleophile is a silane compound of formula R²(R³)₂SiH, R² and R³ being independently from each other selected from (C₁-C₆)alkyl, (C₁-C₆)alkoxy, and (C₆-C₁₀)aryl groups, said aryl groups being optionally substituted with at least one substituent selected from: (C₁-C₆)alkyl, (C₁-C₆)alkoxy, -CN, -OCF₃, -NRₐR_{b}, -Si(R_{c})₃, -BRₐR_{b}, (C₂-C₆)alkenyl, (C₂-C₆)alkynyl, -C(=O)-NRₐR_{b}, -C(=O)-O(C₁-C₆)alkyl, and CF₃, Rₐ, R_{b} and R_{c} being as defined above.

In one embodiment of the process according to the invention, the nucleophile is a silane compound of formula R²(R³)₂SiH, R² and R³ being independently from each other selected from (C₁-C₆)alkyl, (C₁-C₆)alkoxy, and (C₆-C₁₀)aryl groups, said aryl groups being optionally substituted with at least one substituent selected from: (C₁-C₆)alkyl, (C₁-C₆)alkoxy, and -C(=O)-O(C₁-C₆)alkyl, and CF₃.

In one embodiment of the process according to the invention, the nucleophile is selected from the group consisting of: EtMe₂SiH, Me(OMe)₂SiH, Me(OEt)₂SiH, (OEt)₃SiH, Et₃SiH, tBuMe₂SiH, Me₂PhSiH, Me₂BnSiH, MePh₂SiH, Ph₃SiH, Me₂(*p-*CF₃(C₆H₄))SiH, and Me₂(*p*-tBu(C₆H₄))SiH.

In one embodiment, the nucleophile is Me₂(*p*-CF₃(C₆H₄))SiH or Me₂BnSiH, and preferably the nucleophile is Me₂(*p*-CF₃(C₆H₄))SiH.

According to one embodiment, in the process according to the invention, the ligand is selected from the group consisting of: 1,5-cyclooctadiene (COD), 1,2-Bis(dicyclohexylphosphino)ethane (dcype), 1,1'-Bis(diphenylphosphino)ferrocene (dppf), 1,1'-Bis(di-*tert*-butylphosphino)ferrocene (DTBPF), 4,5-Bis(diphenyl-phosphino)-9,9-dimethylxanthene (XantPhos), and triphenylarsine (AsPh₃).

Preferably, the metallic complex is a Ni(0) complex such as bis(1,5-cyclooctadiene)nickel.

According to one embodiment, the solvent used in the reaction of the compound of formula (I) with the nucleophile according to the invention is selected from the usual solvents used in the field. Preferably, the solvent for this step according to the invention is toluene.

According to one embodiment of the process according to the invention, the reaction of the compound of formula (I) with the nucleophile is carried out a temperature from 90°C to 140°C. Preferably, this reaction may be carried out at 90°C, 120°C, and 140°C.

According to one embodiment, in formula (I) as defined above, A is selected from the heteroaryl groups comprising from 5 to 10 atoms and including at least one heteroatom selected from O, N, and S, said heteroaryl groups being optionally substituted with at least one substituent selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above.

The present invention also relates to the process as defined above, wherein the compound of formula (I) is a compound having the following formula (I-1):

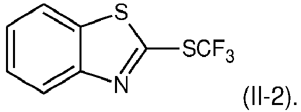

### EXAMPLES

### Example 1: with secondary anilines

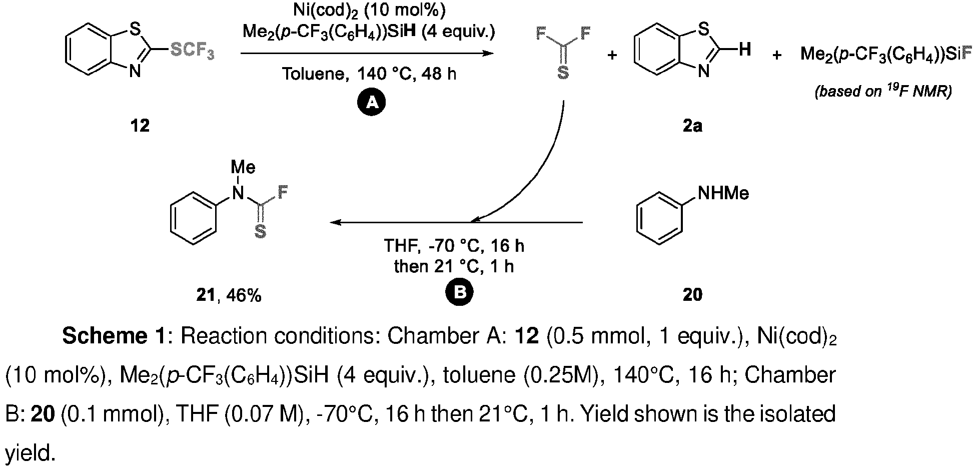

An oven-dried two-chamber tubes both equipped with a stirring bar (chamber A: 10 mL and chamber B: 2 mL) connected by a reflux condenser was filled as follow: Ni(cod)₂ (14 mg, 0.05 mmol, 10 mol%), 2-((trifluoromethyl)thio)benzo[*d*]thiazole 12 (118 mg, 0.5 mmol, 1 equiv.), Me₂(*p*-CF₃(C₆H₄))SiH (408 mg, 2 mmol, 4 equiv.) and toluene (2 mL) under argon for the chamber A and with secondary aniline (0.1 mmol) and THF (1.5 mL) under argon for the chamber B. The resulting solutions were stirred at 140 °C for 16 h (chamber A) and at -70 °C for 16 h then at 21 °C for 1 h (chamber B). The residue of the chamber B was concentrated under vacuum and purified by silica gel flash column chromatography by dry loading the samples and eluting with a solvent system as noted below to afford the desired products.

### Example 2: with alkyl alcohols

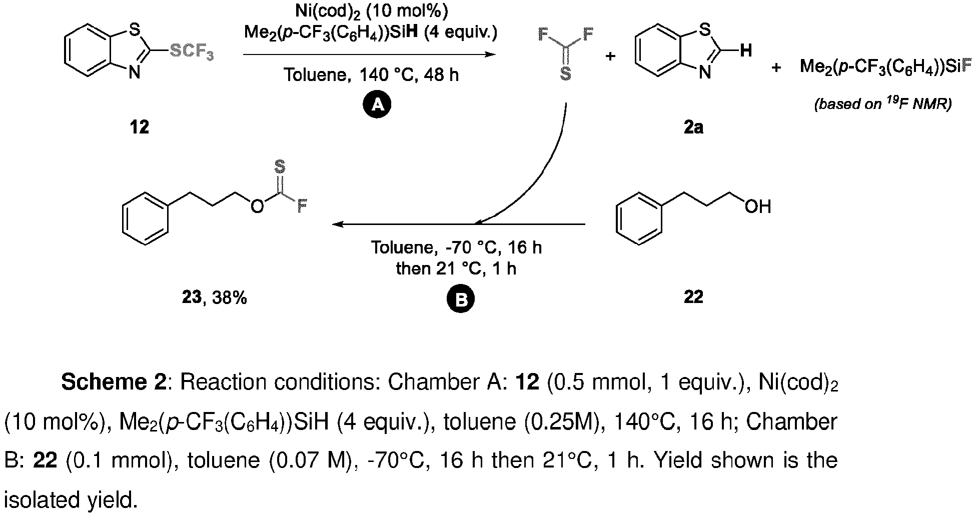

An oven-dried two-chamber tubes both equipped with a stirring bar (chamber A: 10 mL and chamber B: 2 mL) connected by a reflux condenser was filled as follow: Ni(cod)₂ (14 mg, 0.05 mmol, 10 mol%), 2-((trifluoromethyl)thio)benzo[d]thiazole 12 (118 mg, 0.5 mmol, 1 equiv.), Me₂(*p*-CF₃(C₆H₄))SiH (408 mg, 2 mmol, 4 equiv.) and toluene (2 mL) under argon for the chamber A and with alkyl alcohol (0.1 mmol) and toluene (1.5 mL) under argon for the chamber B. The resulting solutions were stirred at 140 °C for 16 h (chamber A) and at -70 °C for 16 h then at 21 °C for 1 h (chamber B). The residue of the chamber B was concentrated under vacuum and purified by silica gel flash column chromatography by dry loading the samples and eluting with a solvent system as noted below to afford the desired products.

## Claims

1. A process for trapping SCF₂ comprising the following steps:
- a step of reacting a compound having the formula (I)
A-SCF₃ (I)
with a nucleophile, in the presence of a metallic complex comprising nickel, a ligand, and a solvent,
A being selected from the group consisting of:
. (C₆-C₁₀)aryl groups,
said aryl groups being optionally substituted with at least one substituent preferably selected from the group consisting of: halogen, (C₁-C₆)alkyl, (C₁-C₆)alkoxy, -CN, -CF₃, -OCF₃, -NRₐR_{b}, -SiRₐR_{b}R_{c}, -BRₐR_{b}, (C₂-C₆)alkenyl, (C₂-C₆)alkynyl, -C(=O)-NRₐR_{b}, and -C(=O)-O(C₁-C₆)alkyl;
Rₐ and R_{b} being independently from each other H or a (C₁-C₆)alkyl group;
R_{c} being a (C₁-C₆)alkyl group;
. heteroaryl groups comprising from 5 to 10 atoms and including at least one heteroatom selected from O, N, and S,
said heteroaryl groups being optionally substituted with at least one substituent preferably selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
. a vinyl compound having the following formula (III): 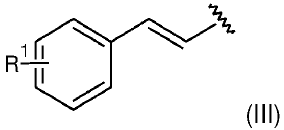 R¹ being selected from the group consisting of: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
said step leading to the formation of a compound AH and SCF₂, and
- a step of reacting SCF₂ with a compound RH, R being a hydrocarbon group, comprising optionally at least one heteroatom,
for obtaining a compound having the formula R-C(=S)-F.

2. The process of claim 1, wherein the compound RH is selected from the group consisting of: amines, alcohols, thiols, phosphines, phosphonates, Grignard reagents, R'Li, selenols, R'₂B(OH), and R'₃SiOH, R' representing an optionally substituted alkyl, aryl, vinyl, or alkynyl group.

3. The process of claim 1 or 2, wherein the compound RH is selected from the group consisting of: secondary alkyl amines, anilines, alkyl alcohols, alkyl thiols, aryl thiols, aryl selenols, phosphine oxides, aryl phosphines, and Grignard reagents.

4. The process of any one of the preceding claims, wherein the compound RH is selected from the group consisting of: HNR_{d}Rₑ, and OR_{f}, R_{d} and Rₑ being independently selected from the group consisting of: (C₁-C₆)alkyl groups and (C₆-C₁₀)aryl groups; and R_{f} being a (C₁-C₆)alkyl group or a (C₆-C₁₀)aryl(C₁-C₆)alkyl group.

5. The process of any one of the preceding claims, wherein the nucleophile is a silane compound of formula R²(R³)₂SiH, R² and R³ being independently from each other selected from (C₁-C₆)alkyl, (C₁-C₆)alkoxy, and (C₆-C₁₀)aryl groups, said aryl groups being optionally substituted with at least one substituent selected from: (C₁-C₆)alkyl, (C₁-C₆)alkoxy, -CN, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, and CF₃, Rₐ, R_{b} and R_{c} being as defined in claim 1.

6. The process of any one of the preceding claims, wherein the nucleophile is selected from the group consisting of: EtMe₂SiH, Me(OMe)₂SiH, Me(OEt)₂SiH, (OEt)₃SiH, Et₃SiH, tBuMe₂SiH, Me₂PhSiH, Me₂BnSiH, MePh₂SiH, Ph₃SiH, Me₂(*p-*CF₃(C₆H₄))SiH, and Me₂(*p*-tBu(C₆H₄))SiH.

7. The process of any one of the preceding claims, wherein the nucleophile is Me₂(*p*-CF₃(C₆H₄))SiH or Me₂BnSiH.

8. The process of any one of the preceding claims, wherein the nucleophile is Me₂(*p*-CF₃(C₆H₄))SiH.

9. The process of any one of the preceding claims, wherein the ligand is selected from the group consisting of: 1,5-cyclooctadiene (COD), 1,2-Bis(dicyclohexylphosphino)ethane (dcype), 1,1'-Bis(diphenylphosphino)ferrocene (dppf), 1,1'-Bis(di-*tert*-butylphosphino)ferrocene (DTBPF), 4,5-Bis(diphenyl-phosphino)-9,9-dimethylxanthene (XantPhos), and triphenylarsine (AsPh₃).

10. The process of any one of the preceding claims, wherein the solvent is toluene.

11. The process of any one of the preceding claims, wherein the reaction of the compound of formula (I) with a hydride is carried out at a temperature from 90°C to 140°C.

12. The process of any one of the preceding claims, wherein the metallic complex is a Ni(0) complex such as bis(1,5-cyclooctadiene)nickel.

13. The process of any one of the preceding claims, wherein A is selected from the heteroaryl groups comprising from 5 to 10 atoms and including at least one heteroatom selected from O, N, and S, said heteroaryl groups being optionally substituted with at least one substituent selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined in claim 1.

14. The process of any one of the preceding claims, wherein the compound of formula (I) is a compound having the following formula (I-1):
